# EUROPÄISCHE PATENTANMELDUNG

(11) **EP 1 374 883 A1**
(43) Veröffentlichungstag der Anmeldung: **02.01.2004**
(21) Anmeldenummer: 02013782.4
(22) Anmeldetag: 21.06.2002
(51) Int. Cl.: A61K 35/78, A61K 9/12, A61P 31/22, A61P 9/02, A61P 29/00

(54) **Nasenspray enthaltend ein Pflanzenextrakt zur Behandlung von Krankheiten wie Herpes Zoster, Blutergüssen, Mastitis und Kreislaufschwäche**

(71) Anmelder: Lingg Roland. Dr., 88299 Leutkirch (DE)
(72) Erfinder: Lingg Roland. Dr., 88299 Leutkirch (DE)
(74) Vertreter: Kiessling, Christian

(57) **Zusammenfassung**

Bei einem Verfahren zur Erregung der Chemorezeptoren des menschlichen Gehirnes mittels einer vorgegebenen Substanz wird ein wirksames Einbringen von Wirksubstanzen in das Gehirn dadurch erreicht, dass die Substanz in Wasser oder einem öligen Fett oder in Alkohol gelöst ist und die Lösung auf die Nasenriechschleimhäute aufgebracht wird.

## Beschreibung

Die Erfindung betrifft ein Mittel zur Erregung der Chemorezeptoren des menschlichen Gehirnes. Des weiteren betrifft die Erfindung ein Verfahren zur Erregung der Chemorezeptoren des menschlichen Gehirnes mittels einer vorgegebenen Substanz.

Das Einbringen von Medikamenten in das menschliche Gehirn zur Behandlung beispielsweise der Parkinsonschen oder Alzheimerschen Krankheit ist mit Schwierigkeiten verbunden, da die Wirksubstanzen der Medikamente sowohl bei oraler Einnahme als auch bei intravenöser Applikation aufgrund einer besonderen Ausbildung des Endothels der Gehirngefäße, der sogenannten Blut/Hirn-Schranke, nicht oder nur zu einem sehr geringen Prozentsatz zu ihrem Bestimmungsort im Gehirn gelangen. Dieses allgemeine Problem lässt sich herkömmlicher weise allein dadurch umgehen, dass die betreffenden Wirksubstanzen in sehr hoher Konzentration verabreicht werden, wobei verstärkte Nebenwirkungen in Kauf zu nehmen sind.

Aufgabe der Erfindung ist es deshalb, ein Mittel und ein Verfahren zu schaffen, mittels derer Wirksubstanzen wirksamer als über die Blutbahn in das Gehirn einbringbar sind.

Für das Mittel der eingangs genannten Art wird diese Aufgabe dadurch gelöst, dass eine Wirksubstanz in Wasser oder in einem fetten Öl oder in Alkohol gelöst auf die Nasenriechschleimhäute aufgebracht wird.

Für das Verfahren der eingangs genannten Art wird diese Aufgabe dadurch gelöst, dass die Substanz in Wasser oder in einem fetten Öl oder in Alkohol gelöst ist und die Lösung auf die Nasenriechschleimhäute aufgebracht wird.

Bevorzugte Ausführungsformen der Erfindung sind Gegenstand der Unteransprüche.

Bei dem erfindungsgemäßen Verfahren und dem erfindungsgemäßen Mittel wird durch das Merkmal, dass eine vorgegebene Wirksubstanz in Wasser oder in einem fetten Öl oder in Alkohol gelöst auf die Nasenriechschleimhäute aufgebracht wird, erreicht, dass die Wirksubstanz von der Nasenriechschleimhaut direkt in die olfaktorisch empfindlichen und mit entsprechenden Chemorezeptoren versehenen Gehirnbereiche wie dem Mandelkern und der Amygdala gelangt. Dort löst dann die Wirksubstanz selbst oder auch eine in der räumlichen Anordnung der Moleküle der Wirksubstanz enthaltene Information die gewünschte Reaktion aus. Diese Reaktion kann beispielsweise in einer verstärkten Durchblutung eines bestimmten Hirnareals oder eines bestimmten Organs bestehen, oder sie kann in einer Ausschüttung von Botenstoffen oder Hormonen aus endokrinen Drüsen bestehen.

Gemäß einer ersten bevorzugten Ausführungsform des erfindungsgemäßen Verfahrens ist vorgesehen, dass die Lösung als Spray mit einem besonders hohen Austrittsdruck von etwa 1500 mBar bis 3000 mBar, vorzugsweise etwa 2000 mBar verabreicht wird. Dadurch ist insbesondere die Möglichkeit geschaffen, die Lösung auf die oberhalb von die Funktion des Befeuchtens und Reinigens von Atemluft erfüllenden Nasenschleimhautteilen gelegene Nasenriechschleimhaut zu transportieren. Die Nasenriechschleimhaut ist dabei im menschlichen Schädel in unmittelbarer Nachbarschaft der zentralen Gehirnareale lokalisiert und steht mit diesen über Nervenbahnen in einer Kommunikationsverbindung.

Gemäß einer bevorzugten Ausführungsform des erfindungsgemäßen Verfahrens ist deshalb vorgesehen, dass die Substanz auf die olfaktorischen Chemorezeptoren der Nasenriechschleimhaut aufgebracht wird und über die olfaktorischen Chemorezeptoren der Nasenriechschleimhaut in den Zerebralraum eindiffundiert und über den Liquor des Gehirnes an zentrale Hirnareale gelangt.

Gemäß einer alternativen bevorzugten Ausführungsform des erfindungsgemäßen Verfahrens ist vorgesehen, dass die Substanz auf die olfaktorischen Chemorezeptoren der Nasenriechschleimhaut aufgebracht wird, wobei die Substanz eine vorgegebene Information trägt, die von den olfaktorischen Chemorezeptoren der Nasenriechschleimhaut wahrnehmbar ist und über neuronale Verbindungen an zentrale Hirnareale weitergeleitet wird.

Die gemäß dem erfindungsgemäßen Verfahren applizierte Substanz kann ein Arzneimittel wie ein gängiges allopathisches Arzneimittel, insbesondere jedoch auch ein homöopathisches Arzneimittel sein. Der bei homöopathischen Mitteln oft verwendete hohe Verdünnungsgrad einer Wirksubstanz findet dabei seine Entsprechung in der hohen Empfindlichkeit der Nasenriechschleimhaut, die in der Lage ist, geringste Mengen bestimmter Substanzen zu erkennen.

Die Vorteile des erfindungsgemäßen Mittels und ihrer Wirksubstanz entsprechen denen der Anwendung des erfindungsgemäßen Verfahrens.

Das erfindungsgemäße Verfahren und das erfindungsgemäße Mittel beruhen auf folgender Erkenntnis:

Die Afferenzen der Riechschleimhaut führen durchs Siebbein zu synaptischen Glomeruli und im zweiten Neuron in die paläopallische Zielregion des tractus olfactorius und dessen Verschaltung mit Kerngebieten des Zwischenhirns. In diesen Kerngebieten und Funktionskreisen von limbischem System, von Thalamus und Hypothalamus findet sich nach Auskunft moderner Neurobiologie und Neuropsychologie die Repräsentanz vitaler somatischer und psychischer Steuerung. Wenn nun schon der Informationsgehalt von intranasalen Substanzmolekülen in diese zerebralen psychosomatischen Steuerungsräume gelangen kann, dann ist auch der homöopathisch- therapeutische Anspruch an ihre Monopräparate (z.B. Arsenicum album), sie erreichten den Menschen gleichermaßen in körperlicher und seelischer Weise, im olfaktorischen Modell vorstellbar und auf wissenschaftlichem Niveau thematisierbar.

Zwei Arten der Chemorezeption sind aus der HNO bekannt: die gustatorische über den Apparat der Geschmacksknospen und die olfaktorische, deren etwa 100 Millionen Rezeptoren in der Riechschleimhaut der regio olfactoria sitzen. Das Riechepithel besteht pro Nasenhälfte aus zwei etwa pfenniggroßen gelblichbraunen Schleimhautfeldern im Bereich der oberen Siebbeinmuschel und der gegenüberliegenden Nasenscheidewand. Insgesamt lassen sich etwa 500 MM2 Ausdehnung und eine Dicke des Epithels von etwa 0, 06 mm annehmen.

In der olfaktorischen Schleimhaut finden sich Trigeminusendigungen, Schleimdrüsen (Bowman'sche Drüsen), Stützzellen und Riechzellen. Letztere sind bipolare Nervenzellen mit einem relativ kurzen, distalen rezeptorischen Dendrit und einem längeren zentripetalen Axon.

Am distalen Ende eines jeden Axons sitzt der sogenannte Riechkegel. Jeder verfügt über etwa zehn Zilien oder Riechgeißein, die in die epitheleale Schleimschicht hineinragen. Mehrere zentripetale Axone treten, umgeben von einer gemeinsamen Schwannzelle, nach kranial durch die Öffnungen der lamina cribrosa des Siebbeins. Diese Axonenbündel (nervi olfactorii) sind die sogenannten Riechfäden.

Die Membran einer bestimmten Riechgeißel kann wahrscheinlich nur eine bestimmte Molekülgröße wahrnehmen, und auch die räumliche Anordnung der Atome innerhalb eines Moleküls der in der Riechschleimhaut gelösten Substanz scheint rezeptorisch erfasst zu werden. Bei etwa 100 Millionen Rezeptoren kann die Feinheit der Erfassung nur geahnt werden. Nach ihrem Eintritt in den bulbus olfactorius bilden jeweils 25.000 Axone präsynaptische Netze, denen postsynaptisch insgesamt etwa 50.000 Mitralzellendendriten und 150.000 Büschelzellen entsprechen. Diese Synapsen zwischen erstem und zweitem Neuron werden Glomeruli genannt. Man kann von etwa 40.000 Glomeruli ausgehen.

Wie bereits die vielen Rezeptoren in der Schleimhaut sich spezifisch auf bestimmte Substanzen "verstehen", so setzt sich eine akzeptierende Selektivität im Bereich der Glomeruli fort. Die Glomeruli verfügen über ein bestimmtes Reaktionsmuster für verschiedene Substanzen. Dieses steht in unmittelbarem Zusammenhang mit den physikalischchemischen Eigenschaften der jeweiligen Geruchsstoffmoleküle. Ein Beispiel aus der Tierwelt gibt Frotscher, wenn er einem substanzspezifischen Rezeptor der Mäuseriechschleimhaut lediglich zwei der vorhandenen 1800 Glomeruli zuordnet.

Die Axone der Mitralzellen, also Axone der zweiten Neuronen der Riechbahn, ziehen als dreikantiger Tractus olfactorius, der in der etwa 4 cm langen Olfäktoriusfurche des basalen Stirnlappens gelagert ist, zu den Riechzentren. Zwei markweiße Streifen sind sichtbar: die stria medialis und die stria lateralis, bisweilen auch eine stria intermedius.

Der mediale Strang der Riechbahn endet im tuberculum olfactorium und dem Nucleus olfactorius anterior, wo er Anschluß ans *limbische System* erhält, unter anderem an Kerne des medialen Thalamusbereich und an den Hypothalamus. Die stria olfactoria lateralis endet im Cortex praepirifortnis bzw. im Nucleus corticalls des Amygdalums.

Die wichtigste Efferenz des Corpus amygdaloldeum ist die Stria tenninalis, die unter anderem zu den Kernen des Hypothalamus führt. Dass dieser die zentrale Region für die Steuerung der vegetativen Funktionen darstellt, ist bekannt. Auch, dass er über seine Verbindungen zu Hypophyse das endokrinvaskuläre System wesentlich beeinflusst. Zu guter letzt spielen hypothalamische Erregungen beim Zustandekommen von Affektionen wie Lustunlustgefühlen, Freude, Angst oder Wut eine große Rolle.

Die hier skizzierten neuroanatomischen Räume und Verschaltungen repräsentieren wesentliche Anteile dessen, was man die somatopsychische Funktionsebene des Menschen nennen könnte. Da die olfaktorischen Afferenzen wie oben beschrieben innige Verbindungen und Verschaltungen innerhalb des limbischen Systems eingehen, dann erklärt es sich zwanglos, warum Geruchsempfindungen so eng mit emotional- effektiven und vegetativen Reaktionen gekoppelt sind.

Das chemorezeptorische Modell propagiert einen Transfer jenseits der Blutwege. Es benennt als ersten Empfänger des Medikamentes präzise das olfaktorische Sinnesorgan in der regio olfactoria der Nase. Wie die obigen Ausführungen zeigen, ist dieses Organ wie geschaffen sowohl für die Diversität insbesondere möglicher homöopathischer Einzelsubstanzen wie auch für deren höchst subtile Verdünnungen bzw. Potenzierungen.

Das erfindungsgemäße Mittel wird im folgenden anhand bevorzugter Ausführungsformen erläutert:

In folgenden Fällen wurden herkömmliche apothekenpflichtige Arzneimittel in gelöster Form bei einer Verwendung als Nasenspray regelmäßig mit Erfolg verabreicht. Die Bezeichnung eines Mittels (z.B. Arnica) entspricht dabei dem in Alkohol oder Wasser gelösten Inhaltsstoff. Die Bezeichnung D1 bedeutet dabei eine Verdünnung im Verhältnis 1/10.
- **Beispiel 1**
- Mezereum D4 in Alkohol oder Wasser gelöst und als Nasenspray mit einem Druck von 2000 bar appliziert heilt ein Herpes zoster (viraler Nervenbefall) aus.
-
- **Beispiel 2**
- Arnica D6 in Alkohol oder Wasser gelöst und als Nasenspray mit einem Druck von 2000 bar appliziert bewirkt die Rückbildung von Blutergüssen nach traumatischer Contusio (Prellungen, Stauchungen).
-
- **Beispiel 3**
- Bryonia D8 in Alkohol oder Wasser gelöst und als Nasenspray mit einem Druck von 2000 bar appliziert bewirkt das Abklingen einer schmerzhaften Mastitis (Brustentzündung) bei einer stillenden Frau.
-
- **Beispiel 4**
- Veratrum album D8 in Alkohol oder Wasser gelöst und als Nasenspray mit einem Druck von 2000 bar appliziert behebt eine Kreislaufschwäche mit Kaltschweißigkeit und Vertigo (Schwindel).

Die oben erläuterten Ausführungsbeispiele der Erfindung dienen lediglich dem Zweck eines besseren Verständnisses der durch die Ansprüche definierten erfindungsgemäßen Lehre, die als solche durch die Ausführungsbeispiele nicht eingeschränkt ist.

## Patentansprüche

**1.** Verfahren zur Erregung der Chemorezeptoren des menschlichen Gehirnes mittels einer vorgegebenen Substanz, **dadurch gekennzeichnet, dass** die Substanz in Wasser gelöst ist und die Lösung auf die Nasenriechschleimhäute aufgebracht wird.

**2.** Verfahren zur Erregung der Chemorezeptoren des menschlichen Gehirnes mittels einer vorgegebenen Substanz, **dadurch gekennzeichnet, dass** die Substanz in einem fetten Öl gelöst ist und die Lösung auf die Nasenriechschleimhäute aufgebracht wird.

**3.** Verfahren zur Erregung der Chemorezeptoren des menschlichen Gehirnes mittels einer vorgegebenen Substanz, **dadurch gekennzeichnet, dass** die Substanz in Alkohol gelöst ist und die Lösung auf die Nasenriechschleimhäute aufgebracht wird.

**4.** Verfahren nach einem der Ansprüche 1 bis 3, **dadurch gekennzeichnet, dass** die Lösung als Spray mit einem Austrittsdruck von etwa 1500 mBar bis 3000 mBar verabreicht wird.

**5.** Mittel nach einem der vorhergehenden Ansprüche, **dadurch gekennzeichnet, dass** die Substanz über die olfaktorischen Chemorezeptoren der Nasenriechschleimhaut in den Zerebralraum eindiffundiert und über den Liquor des Gehirnes an zentrale Hirnareale gelangt.

**6.** Verfahren nach einem der vorhergehenden Ansprüche, **dadurch gekennzeichnet, dass** die Substanz eine vorgegebene Information trägt, die von den olfaktorischen Chemorezeptoren der Nasenriechschleimhaut wahrnehmbar ist und über neuronale Verbindungen an zentrale Hirnareale weitergeleitet wird.

**7.** Verfahren nach einem der Ansprüche 5 oder 6, **dadurch gekennzeichnet, dass** die Substanz ein Arzneimittel ist.

**8.** Verfahren nach Anspruch 7, **dadurch gekennzeichnet, dass** die Substanz ein allopathisches Arzneimittel ist.

**9.** Verfahren nach Anspruch 7, **dadurch gekennzeichnet, dass** die Substanz ein homöopathisches Arzneimittel ist.

**10.** Mittel zur Erregung der Chemorezeptoren des menschlichen Gehirnes, **dadurch gekennzeichnet, dass** eine Wirksubstanz in Wasser gelöst auf die Nasenriechschleimhäute aufgebracht wird.

**11.** Mittel zur Erregung der Chemorezeptoren des menschlichen Gehirnes, **dadurch gekennzeichnet, dass** eine Wirksubstanz in einem fetten Öl gelöst auf die Nasenriechschleimhäute aufgebracht wird.

**12.** Mittel zur Erregung der Chemorezeptoren des menschlichen Gehirnes, **dadurch gekennzeichnet, dass** eine Wirksubstanz in Alkohol gelöst auf die Nasenriechschleimhäute aufgebracht wird.

**13.** Mittel nach einem der Ansprüche 10 bis 12, **dadurch gekennzeichnet, dass** das Mittel in Form eines Sprays mit einem Austrittsdruck von etwa 1500 mBar bis 3000 mBar verabreicht wird.

**14.** Mittel nach einem der Ansprüche 10 bis 13, **dadurch gekennzeichnet, dass** die Wirksubstanz über die olfaktorischen Chemorezeptoren der Nasenriechschleimhaut in den Zerebralraum eindiffundiert und über den Liquor des Gehirnes an zentrale Hirnareale gelangt.

**15.** Mittel nach einem der Ansprüche 10 bis 13, **dadurch gekennzeichnet, dass** die Wirksubstanz eine vorgegebene Information trägt, die von den olfaktorischen Chemorezeptoren der Nasenriechschleimhaut wahrnehmbar ist und über neuronale Verbindungen an zentrale Hirnareale weitergeleitet wird.

**15.** Mittel nach einem der Ansprüche 13 oder 14, **dadurch gekennzeichnet, dass** die Wirksubstanz ein Arzneimittel ist.

**16.** Mittel nach Anspruch 15, **dadurch gekennzeichnet, dass** die Wirksubstanz ein homöopathisches Arzneimittel ist.

**17.** Mittel nach Anspruch 15, **dadurch gekennzeichnet, dass** die Wirksubstanz ein allopathisches Arzneimittel ist.

**18.** Mittel zu Behandlung von Herpes zoster gemäß dem Verfahren nach einem der Ansprüche 1 bis 9, **dadurch gekennzeichnet, dass** Mezereum D4 in Alkohol gelöst als

**19.** Mittel zu Behandlung von Herpes zoster gemäß dem Verfahren nach einem der Ansprüche 1 bis 9, **dadurch gekennzeichnet, dass** Mezereum D4 in Wasser gelöst als Nasenspray appliziert wird.

**20.** Mittel zu Behandlung von Blutergüssen gemäß dem Verfahren nach einem der Ansprüche 1 bis 9, **dadurch gekennzeichnet, dass** Arnica D6 in Alkohol gelöst als Nasenspray appliziert wird.

**21.** Mittel zu Behandlung von Blutergüssen gemäß dem Verfahren nach einem der Ansprüche 1 bis 9, **dadurch gekennzeichnet, dass** Arnica D6 in Wasser gelöst als Nasenspray appliziert wird.

**22.** Mittel zu Behandlung von Mastitis gemäß dem Verfahren nach einem der Ansprüche 1 bis 9, **dadurch gekennzeichnet, dass** Bryonia D8 in Alkohol gelöst als Nasenspray appliziert wird.

**23.** Mittel zu Behandlung von Mastitis gemäß dem Verfahren nach einem der Ansprüche 1 bis 9, **dadurch gekennzeichnet, dass** Bryonia D8 in Wasser gelöst als Nasenspray appliziert wird.

**24.** Mittel zu Behandlung von Kreislaufschwäche gemäß dem Verfahren nach einem der Ansprüche 1 bis 9, **dadurch gekennzeichnet, dass** Veratrum album D8 in Alkohol gelöst als Nasenspray appliziert wird.

**25.** Mittel zu Behandlung von Kreislaufschwäche gemäß dem Verfahren nach einem der Ansprüche 1 bis 9, **dadurch gekennzeichnet, dass** Veratrum album D8 in Wasser gelöst als Nasenspray appliziert wird.
